# EUROPEAN PATENT SPECIFICATION

(11) **EP 2 992 914 B1**
(45) Date of publication and mention of the grant of the patent: **01.11.2023**
(21) Application number: 14183555.3
(22) Date of filing: 04.09.2014
(51) Int. Cl.: A61M 1/04, A61B 17/34, A61M 27/00, A61M 25/01, A61M 25/06, A61M 25/00, A61M 39/24

(54) **A transcutaneous device for removal of fluid from a body**
Transkutane Vorrichtung zur Beseitigung von Flüssigkeit aus einem Körper
Dispositif trans-cutané pour l'élimination de fluide dans un corps

(43) Date of publication of application: 09.03.2016
(73) Proprietor: Safeguard Medical Technologies Limited, Hope Under Dinmore, Herefordshire HR6 0PW (GB)
(72) Inventor: Russell, Malcolm Quentin, Herefordshire, HR6 0PW (GB); Real, Keith Joseph, Herefordshire, HR6 0PW (GB)
(74) Representative: Haseltine Lake Kempner LLP

(56) References cited:
- EP-A2- 0 943 356
- EP-A2- 1 358 904
- WO-A1-2006/090148
- WO-A1-2009/068661
- WO-A2-02/081013
- AT-A4- 505 614
- DE-A1-102006 052 686
- DE-U1-202013 103 397
- US-A- 4 664 660
- US-A- 5 509 909
- US-A- 5 997 486
- US-A1- 2005 054 983

## Description

### Field of Invention

The invention relates to a device used to treat and manage pneumothoraxes' and effusions through a controlled drainage system while maximising patient safety and minimising the steps required and complexity for the practitioner.

### Background of the Invention

Needle thoracocentesis (NT) is the insertion of a needle into the pleural space and the drainage of air that has accumulated. This can be live-saving when a patient has a tension pneumothorax as it allows decompression which can be vital for the restoration of the circulatory system and for improvement in ventilation.

A needle is inserted into the chest in the 2nd intercostal space in the anterior mid-clavicular line. It is inserted perpendicularly to the chest wall, just above the 3rd rib (to avoid the intercostal neurovascular bundle.

There are many published studies documenting the failure of other devices or improvised cannulas which have been seen to not treat the condition adequately or are simply not long enough to reach the pleural cavity and be effective (Refs 1-5).

Much evidence is now available that indicates using an inappropriate product to perform this procedure may lead to complications or indeed simply be inadequate. Some studies have shown a failure rate in the prehospital environment of as high as 40% (Ref 1). The length of the needle/catheter used can be too short to reach the pleural space to release air/fluid and/or the needle/catheter does reach the pleural space when pressure is applied allowing some release of gas and fluid but the catheter retracts into the intercostal muscle once the needle is withdrawn. In either scenario, this can lead to an NT failure. This is of particular interest to the military as Harcke et al. (Ref 5) have shown chest wall thickness in military personnel to be larger and therefore requiring longer cannula products.

The cannulas used in current NT's are also prone to blockage through kinking of the cannula. This is clinically very significant as in such a case for example a relieved tension pneumothorax may re-accumulate undetected. Many published examples discuss the potential detrimental effects of cannula kinking particularly in cases of tension pneumothorax (Refs 6-8). Jones & Hollingsworth (Ref 6) discuss how in a tension pneumothorax chest compression can cause cannulas to kink and hence occlude, and also that was possible that the nature of cannulas used in the case studies discussed predisposed them to kinking after they had passed through the chest wall and the trocar has been removed. The three cases discussed showed that there is a potential for failure of the NT due to the cannula. Groves and Parekh (Ref 7) discuss how in a case the patient became dyspnoeic and hypoxic post NT due to the cannula kinking or being blocked another way. Post NT procedure life threatening deterioration was believed to be due to haemothorax, pneumothorax or both. The patient continued to deteriorate and then had an asystolic cardiac arrest although no specific autopsy evidence could attribute the death to the NT.

NT is a fifesaving procedure, which involves placing a cannula into the second intercostal space midclavicular line just above the third rib. Surrounding this landmark are the mediastinal structures and the internal mammary artery medially, and the subclavian vessels and subcostal neurovascular bundles superiorly. There have been several case reports of life threatening iatrogenic injury following laceration of these structures during needle thoracocentesis (Refs 9-12). While it is not always possible to exactly determine the cause of a haemothorax it is widely considered a significant issue that most NT products or improvised cannulas have sharp unprotected ends that can cause bleeding through laceration of blood vascularisation in the areas of insertion. Even more significantly if misdiagnosis of a pneumothorax occurs there is a strong possibility of lung laceration with the needle if no pneumothorax is present. Air embolism through such a laceration is also a major concern.

There are a number of devices that are used for NT's currently. The ARS Needle 14 gauge x 3.25 in consists of a sharp trocar to gain entry through the skin and tissues and a cannula tube to allow for decompression. However, it has an unprotected sharp end which could cause lung laceration or laceration of blood vascularisation and the cannula also kinks easily which could lead to cannula occlusion and the effects that can cause. The length of the product also means that it may also be too short to reach the pleural space in some individuals.

The ThoraQuik product is another decompression needle product being a cannula of 10 cm in length. A Veress needle type tip is atraumatic to lung tissue and blood vascularisation. However, the cannula kinks easily and the device cannot be inserted in a single operation. The device requires that a scalpel be used to cut skin before it can be used which limits its use in the prehospital environment where paramedic staff do not always carry scalpels and are usually precluded from using scalpels in some territories. There are other such decompression needles available but all have one or more of the significant issues described herein.

There is therefore a need for a device that overcomes the above-mentioned disadvantages of current NT devices and improvised devices.

AT505614A (US2010/0268156A) describes a catheter device for supplying fluid to and removing fluid from body cavities. The catheter has a valve at the proximal end through which a veress needle is inserted.

### Summary of the Invention

According to the invention there is provided a single step body insertion device for removal of fluid from a body as set out in claim 1 below.

In one embodiment the main body of the cannula shaft is of a uniform thickness and the wall thickness progressively reduces inwardly distally along the distal tip of the cannula
wherein the cannula shaft is of a polymeric material and is flexible and kink resistant.

In one embodiment the main body of the cannula shaft is of a uniform thickness and the wall thickness progressively reduces inwardly distally along the distal tip of the cannula.

The inner diameter (IDt) along the length of the tapered tip may be less than the inner diameter (IDb) of the main body of the cannula shaft. The difference between IDb and IDt is less than 0.4mm, preferably less than 0.2mm, preferably approximately 0.16mm.

In one embodiment the cannula shaft is of a polymeric material with a shore hardness of about 63D. The cannula shaft may be of an aliphatic polyether-based thermoplastic polyurethane. The cannula shaft may be radiopaque. The shaft may contain a radiopaque material. The radiopaque material may be barium sulphate. Barium sulphate may be present at a loading of about 20% by weight in the cannula of the device.

In one embodiment the taper angle of the outer surface of the distal tip is from 4° to 13°. The taper angle may be from 7° to 11°. The taper angle may be approximately 9°.

In one embodiment the length of the tapered distal tip is less than 10mm. The length of the tapered distal tip is about 4mm.

In one case the length of the cannula shaft is at least 100mm. The length of the cannula shaft may be from 100 to 150mm. In one case the length of the cannula shaft is about 115mm.

In one embodiment the cannula shaft has an outer diameter in the range of from 2 to 5mm. The cannula shaft may have an outer diameter in the range of from 3 to 4mm. The cannula shaft may have an outer diameter of about 3.5mm.

In one embodiment the main body of the cannula shaft has a wall thickness in the range of from 0.5mm to 1mm. The main body of the cannula shaft may have a wall thickness of about 0.7mm.

In one embodiment the main body of the cannula shaft has at least one opening in the wall thereof adjacent to the distal tip to provide a further pathway for fluid entry into the cannula shaft.

In one case the cannula shaft has indicia thereon to indicate the depth of penetration of the cannula shaft into the body. At least some of the indicia may be luminous.

In one embodiment the side port comprises a connector for mounting of ancillary components to the side port. The connector may be a Luer type connector.

In one embodiment one-way valve is a low pressure cracking valve having a cracking pressure of ≤ 12mbar and a backward pressure of up to 6 bar.

The device may further comprise a Veress needle which extends through the sealing valve in the hub.

The device may further comprise a three way tap for attachment to the side port. There may be a syringe for attachment to one of the ports of the three way tap.

The invention provides for the management of pneumothoraxes and effusions for both the prehospital and hospital medical practitioner. The device provides advantages over other types of devices used in such settings by incorporating multiple functionalities into one device. Needle thoracostomy and medical drainage functionalities are provided in one unique simple to use device.

The transcutaneous device comprises a large gauge custom tipped cannula attached to a hub with a moulded collar, housing a self-sealing valve which acts as a Veress needle entry point. The hub also contains a side port which comprises a one way low cracking pressure valve.

The device allows fluid and gases to be released from the insertion site thereby providing the user a simple single device procedure to manage a number of conditions that require release of fluid or gas from the body.

In one embodiment the device comprises a one way low pressure cracking valve that has a cracking pressure of ≤ 12 mbar with a backward pressure of up to 6 bar thereby allowing fluid and gases to travel one way but not the other.

In one embodiment the device hub contains a collar section which houses a self-sealing valve which allows for Veress needle removal from the device with subsequent airtight sealing of the Veress needle insertion site to the device.

The cannula is preferably made from a biocompatible flexible and non-kinking polymeric material that is radiopaque.

The cannula is sufficiently flexible to pig tail following Veress needle withdrawal. The cannula also has fenestrations that allow for full fluid and gas functionality even if the primary cannula becomes obstructed.

The cannula skin insertion distal end is tapered at an angle that allows for easy skin penetration without need for skin precutting devices or any other pretreatment processes.

In one embodiment the length of the cannula is greater than 10 cm.

In one case the device is attached to a port of a standard three way tap through the device side port with a syringe attached to a further port of the three way tap. This set-up allows fluids and/or gases to be withdrawn by use of the syringe.

A low pressure cracking valve may be attached to a port of the three way tap in addition to the attachment of the device through its side port cracking valve with the syringe attached to the final port. This set-up has the major advantage that pulling back the plunger on the syringe with subsequent pushing of the plunger converts the device into a highly effective pump for the removal of fluid or gases from the body.

In some embodiments the device may be used as a temporary chest drain. The device has equivalent functionality of chest drains without potentially many of the complications such as haemothroax or lung lacerations.

The invention provides needle thoracentesis, chest drain and fluid pump functionality in a single device that can be inserted by a single practitioner in seconds and without the use of other devices for medical practitioners, the device is intuitive and cross-procedurally efficacious through many procedures where it is necessary to remove fluid or gas from the body.

One particular application of the device is in a prehospital environment as it is a single device solution for the management of tension pneumothorax without the need for skin precutting. The device is also suitable for use in the hospital setting to drain effusions and particularly pleural effusions.

Thus, the device is a suitable transcutaneous device to drain unwanted fluid and gases without the need for a skin precutting device which has significant utility in prehospital use while also being able to function in a hospital setting as a drainage device for effusion required treatments. One unique feature of the invention is that the device can integrate the required functionalities of many medical drainage devices along with the functionalities of needle thoracostomy devices into one single simple device without in any way adversely affecting the functions of the device and, in fact, improving on them.

The device of the invention achieves the balance of being able to penetrate the skin, without the need for a scalpel but is sufficiently flexible to be kink resistant. The tip of the cannula is soft and flexible so that it can enter the skin/muscle along with the Veress needle but has sufficient shore hardness to be able to push through the skin/muscle in a smooth fashion without wrinkling.

The invention provides a simple manual device that has needle length sufficient to treat all patients at all insertions sites in all conditions. The device is atraumatic to lung and blood vascularisation and also kink resistant and able to penetrate the skin and muscle tissues without pre-treating skin with implements such as scalpels. The device may also comprise a valve to prevent reoccurrence of conditions such as tension pneumothorax post utilisation.

In one embodiment of the invention the Veress needle has a luer connection at the proximal end of the device that allows for attachment of a syringe or other device, if required.

The Veress needle has a visual indicator that pushes up when the needle is penetrating the harder skin and muscle layers as it is attached to the Veress protective tip. This visual indicator then pops down on penetrating these layers indicating penetration into the softer cavities of the body. The visual indicator may be luminous and/or made of a material that can be seen at night.

In one embodiment of the invention the Veress needle is structured such that, on penetrating into a body area with fluid and/or gas, it has an internal cavity that allows these to escape through it before full insertion of the rest of the device.

In one embodiment of the invention the cannula is manufactured from flexible kink resistant material such as polyurethane. In further embodiments of the invention the cannula may be constructed with polyethylene, polyvinyl chloride or other such polymers.

In a preferred embodiment of the invention the cannula is manufactured from an aliphatic polyether-based thermoplastic polyurethane with a loading of barium sulphate. In a particular embodiment of the invention the cannula is manufactured from Tecoflex EG-65D-B20 poyurethane which is an aliphatic polyether-based thermoplastic polyurethane with a 20% loading of barium sulphate.

In one embodiment the material used to manufacture the cannula has incorporated a 20% loading of barium sulphate to enable the cannula to be detected under X-ray.

In another embodiment the cannula has fenestrations located about 0.5 cm from the tip of the cannula to allow fluid and/or gas to pass through these fenestrations if the primary channel is blocked.

In another embodiment the cannula is graduated and is greater than 10cm in length so as to be able to reach the pleural cavity in the majority of patients.

In a further embodiment the cannula has a tip configuration with at an angle between 4 and 15°.

In one embodiment the cannula can be left in situ in the patient for up to 30 days due to the biocompatible nature of the material used to manufacture it.

In one embodiment of the invention the device has an over moulded hub section linking the cannula to any side ports and other attached components.

In one embodiment the hub section is designed to act a grip for the device to aid with controlled use.

In one embodiment the hub contains a standard luer connection to allow addition of other components.

In a further embodiment the luer connection on the device hub is a side port and a low pressure one way cracking valve is attached.

In another embodiment this a low pressure one way cracking valve is attached and configured in such a way as to let fluid and/or gas out of the device but not back into the device.

In a preferred embodiment the low pressure one way cracking valve connects to the luer connector on the hub of the device and the valve also comprises a luer connector allowing other devices to be attached to it.

In another embodiment of the invention the hub side port luer connection has a syringe attached.

In one embodiment the device hub contains a collar section from which the Veress needle is inserted and removed.

In another embodiment the collar section of the hub contains a valve device that seals around the Veress needle when it is in the device and seals shut behind the Veress needle on needle removal.

In a further embodiment of the invention the collar on the device hub contains an integrated self-sealing valve.

In another embodiment of the invention it is provided in conjunction with a sharps needle safety device.

In one embodiment of the device the device is connected to a standard three way tap through the side port low pressure one way cracking valve.

In another embodiment of the invention the three way tap has a syringe connected to it to allow withdrawal of fluid and/or gas through the device.

In a further embodiment the three way tap also has another low pressure one way cracking valve attached to it as well as the device and the syringe, which in the correct orientation allows pumping of fluid and/or gas from the body through the device and to and external source.

In one embodiment the transcutaneous invention is used for the management of conditions such as pneumothoraxes and pleural effusions as well as other conditions that require release of fluid and/or gas from the body.

In one embodiment of the invention a syringe is connected to the luer connector of the Veress needle in order to withdraw fluid and/or gas from the body through the device in a controlled manner.

In one embodiment of the invention a three way tap, a syringe and another one way low pressure valve are added in order to be able to pump fluid from the body after insertion of the device.

In one embodiment the invention comprises a one way low pressure cracking valve that has a cracking pressure of ≤ 12 mbar with a backward pressure of up to 6 bar thereby allowing fluid and gases to travel one way but not the other.

In one embodiment the cannula is manufactured form flexible kink resistant material such as polyurethane.

In one embodiment of the tip of the flexible kink resistant material is engineered and angled so that it can penetrate the skin and tissue layers required.

In one embodiment the cannula has a tip configuration with at an angle between 4° and 13°.

In one embodiment the cannula contains an agent to make it detectable under X-ray.

In one embodiment the agent used in the cannula to enable its detection is Barium Sulphate at a loading of 20%.

In one embodiment the cannula may be constructed with polyethylene, polyvinyl chloride or other such polymers.

In one embodiment the cannula has fenestrations located in the tip region of the cannula to allow fluid and/or gas to pass through these fenestrations if the primary channel is blocked.

In one embodiment the cannula is graduated and is greater than 10cm in length so as to be able to reach the pleural cavity in the majority of patients.

### Brief Description of the Drawings

The invention will be more clearly understood from the following description of an embodiment thereof, given by way of example only, with reference to the accompanying drawings, in which:

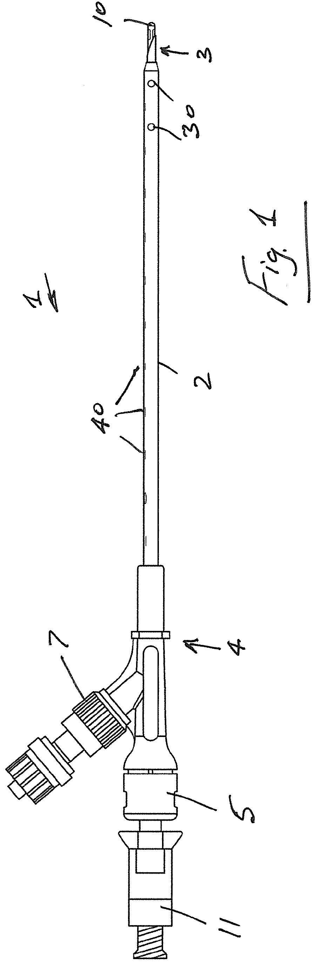

Fig. 1 is an elevational view of a transcutaneous device according to the invention;

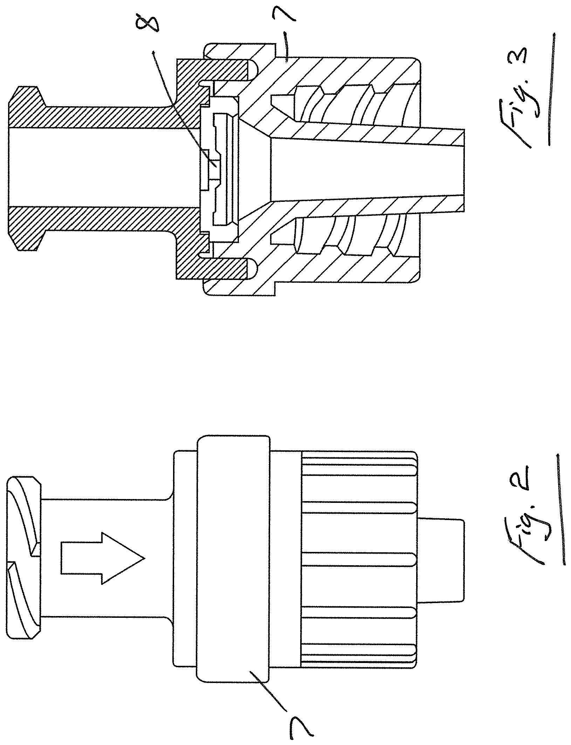

Figs. 2 and 3 are detailed views of a side part with low pressure one way cracking valve of the device;

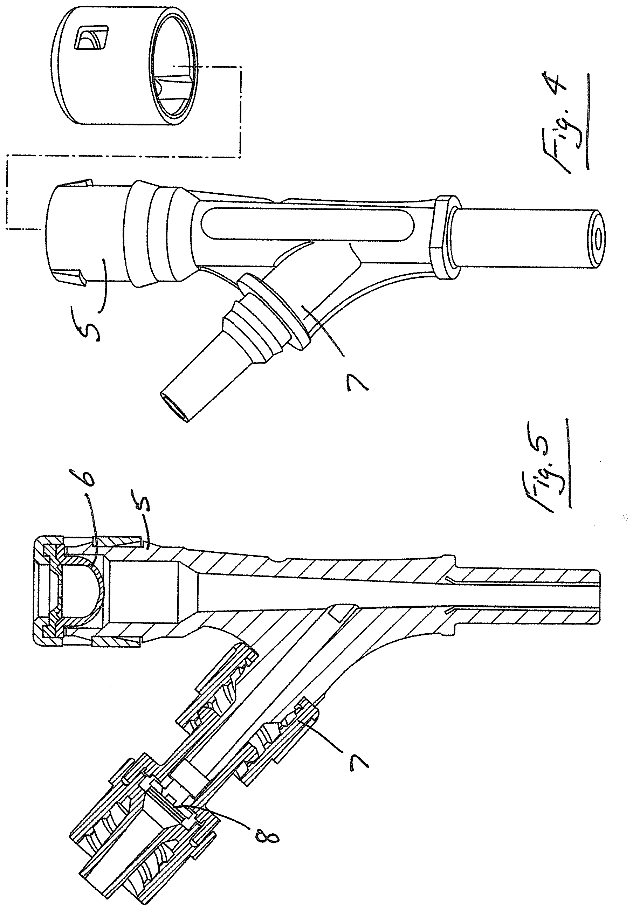

Figs. 4 and 5 are isometric and cross sectional views of a hub section of the device;
Fig. 6 is a cross sectional view of a cannula tip of the device;
Fig. 7 is an enlarged cross section of the tip;
Figs. 8 and 9 Illustrates the insertion site for the device in the treatment of a pneumothorax;
Fig. 10 illustrates the correct insertion method for the device just above the upper border of the third rib (i.e. into the second intercostal space) in the anterior mid-clavicular line, to avoid the intercostal neurovascular bundle for the management of a pneumothorax;
Figs. 11 and 12 illustrate the bending of the shaft of the device, in use;
Fig. 13 illustrates the inserted device with a Veress needle withdrawn and a three way tap attached and a syringe attached to one port of the three way tap;
Fig. 14 illustrates the inserted device with the Veress needle withdrawn and a three way tap attached and a syringe attached to one port of the three way tap and with the final tap closed; and
Fig. 15 illustrates further embodiment of the device including a to Fig. 14 which comprises the addition of low pressure one way cracking valve to the final port of the three way tap.

### Detailed Description

The device of the invention may be used to treat a number of different conditions as follows.
- In a standard pleural effusion an abnormal amount of fluid accumulates around the lung. The excess fluid may accumulate because the body does not handle fluid properly (such as in congestive heart failure, or kidney and liver disease). The fluid in pleural effusions also may result from inflammation, such as in pneumonia, autoimmune disease, and many other conditions.
- In a pneumothorax air accumulates in the pleural space due to a tear of the lung which could occur for a number of reasons including a broken rib penetrating the lung or even simply a sac of the lung spontaneously rupturing.
- A tension pneumothorax standardly occurs in a trauma situation when a continuous leakage of air into the pleural space may occur following for example a penetrating trauma, and this collapses the lung because the air cannot escape. Progressive build-up of pressure in the pleural space pushes the mediastinum to the opposite haemothorax, and obstructs venous return to the heart. This leads to circulatory instability and may result in traumatic arrest. Needle thoracostomy (NT) is the most rapid method of achieving life-saving access to the pleural space.

Referring to the drawings there is illustrated a single step transcutaneous insertion device for removal of fluid from a body. The device comprises a cannula 1 having a cannula shaft 2 with a tapered distal tip 3 and a proximal hub 4. The hub 4 has a housing part 5 with a sealing valve 6 which is aligned with the longitudinal axis of the cannula shaft 2 and a side port 7 with a housing part having a low pressure one way cracking valve 8 which is attached using a standard Luer lock system. In one embodiment the device comprises a one way low pressure cracking valve that has a cracking pressure of ≤ 12 mbar with a backward pressure of up to 6 bar thereby allowing fluid and gases to travel one way but not the other.

Other components may also be attached to the side port using the Luer lock system as will be described in more detail below.

A Veress needle 10 has a proximal end 11 and a needle shaft 12 which extends through the proximal sealing valve 6 and the cannula shaft. In a known manner, the distal end of the needle shaft 12 of the Veress needle 10 is used to puncture the skin surface and relevant tissue layers.

In the invention the cannula shaft 2 is advanced into the body tracking over the needle shaft 12 to reach fluid and/or gases that need to be removed from the body. When the skin has been punctured and the cannula is in place the Veress needle 10 is removed through the sealing valve 6. The sealing valve 6 may be a silicone valve that prevents fluid flow through the valve when the needle is removed.

Body fluid flows up through the cannula shaft to the low pressure one way cracking valve 8 at the cannula side port 7 from which it can be drained and/or removed with the aid of a syringe, if necessary.

The cannula shaft 2 is of a polymeric material which is flexible and kink resistant but has sufficient shore hardness for penetrating through the needle puncture to the site from which fluid is to be removed. The material may be a polyurethane with a shore hardness of about 63D. The cannula shaft is preferably radiopaque. The shaft may contain a radiopacifying agent such as barium sulphate at a loading of about 20% by weight. One such material is Tecoflex EG-65D-B20 polyurethane which is an aliphatic polyether-based thermoplastic polyurethane with a 20% loading of barium sulphate and is available from Lubrizol Corporation, 29400 Lakeland Boulevard, Wickliffe, Ohio 44092, USA.

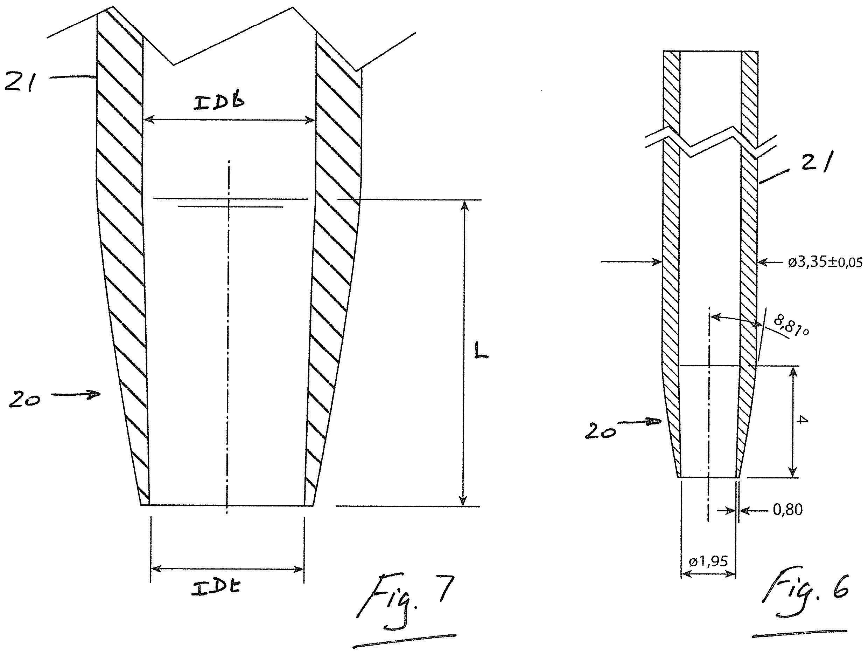

Referring in particular to Figs. 6 and 7 importantly, the cannula shaft 2 has a tapered distal tip section 20 to aid penetration. The main body 21 of the cannula shaft is of a uniform thickness and the wall thickness progressively reduces inwardly distally along the distal tip as will be particularly evident from Fig. 6. We have found that this configuration is optimum to provide the balance of properties required. The taper angle of the outer surface of the tip 20 is from 4° to 13°, preferably 7° to 11° and ideally about 9°. The length of the tip 20 is less than 10mm and is preferably about 4mm. The length of the cannula shaft 2 is preferably at least 100mm, most preferably 100 to 150mm and in one case about 155mm.

The cannula shaft 2 has an outer diameter in the range of from 2 to 5mm, preferably 3 to 4mm with in one case about 3.5mm.

The wall thickness of the cannula shaft main body 21 is in the range 0.5mm to 1mm, preferably about 0.7mm.

The inner diameter (IDt) along at least a portion of, and in this case along the full length of, the distal tip 20 is less than the inner diameter (IDb) of the main body of the cannula shaft. Typically IDb > IDt by an amount of less than 0.4mm, preferably less than 0.2mm, preferably approximately 0.16mm. This is important as it ensures that the distal tip closely hugs the needle shaft whilst allowing the needle to be readily inserted and withdrawn. This IDt provides a resistance aid when the needle and cannula are penetrating the skin so that the cannula can easily follow the needle through the skin.

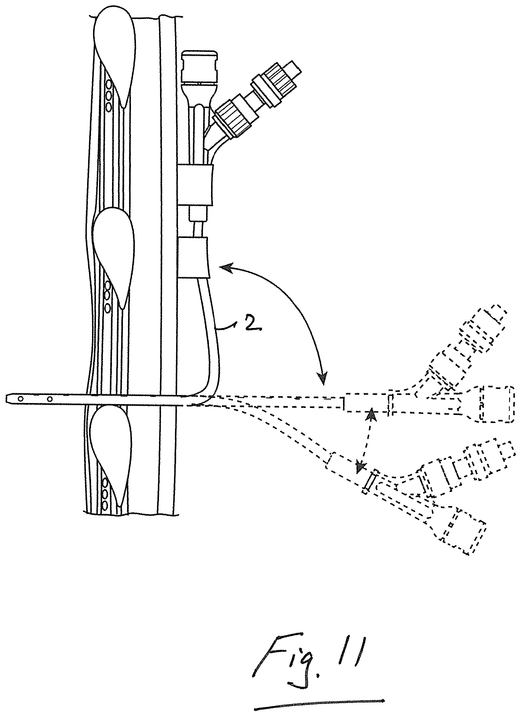

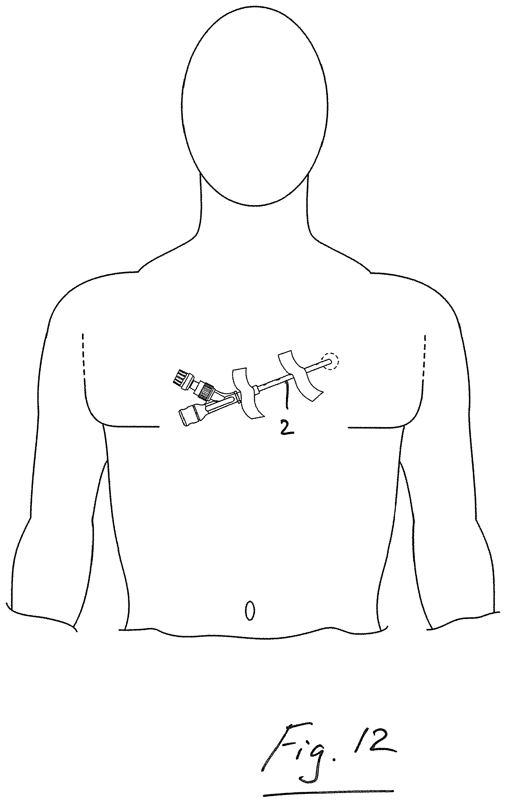

The main body 21 of the cannula shaft 2 has at least one opening 30 in the side wall thereof adjacent to the distal tip to provide a further pathway for fluid entry into the cannula shaft. The cannula is sufficiently flexible to pig tail following withdrawal of the Veress needle 10 and also contains fenestrations 30 that allow for full fluid and gas functionality even if the primary cannula becomes obstructed. The cannula shaft can be maintained as illustrated in Figs. 11 and 12 after withdrawal of the Veress needle and can be taped substantially flat for patient comfort and handling.

The cannula shaft also has indicia 40 marked thereon to indicate the depth of penetration of the cannula shaft into the body. In one embodiment of the invention the Veress needle 11 has a luer connection at the proximal end that allows for attachment of a syringe or other device, if required.

In one embodiment of the invention the Veress needle 10 has a visual indicator that pushes up when the needle is penetrating the harder skin and muscle layers as it is attached to the Veress protective tip. This visual indicator then pops down on penetrating these layers indicating penetration into the softer cavities of the body. The visual indicator may be luminous and/or made of a material that can be seen at night.

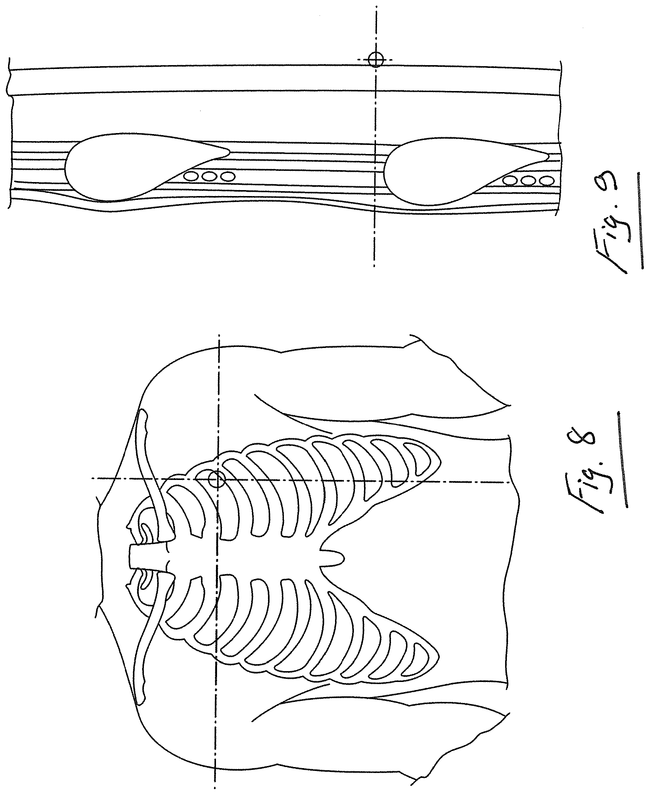

Figs. 8 and 9 show the insertion site for the device in the treatment of a pneumothorax. Following location of which side of the chest the pneumothorax is located the insertion site is just above the upper border of the third rib (i.e. into the second intercostal space) in the anterior mid-clavicular line, to avoid the intercostal neurovascular bundle

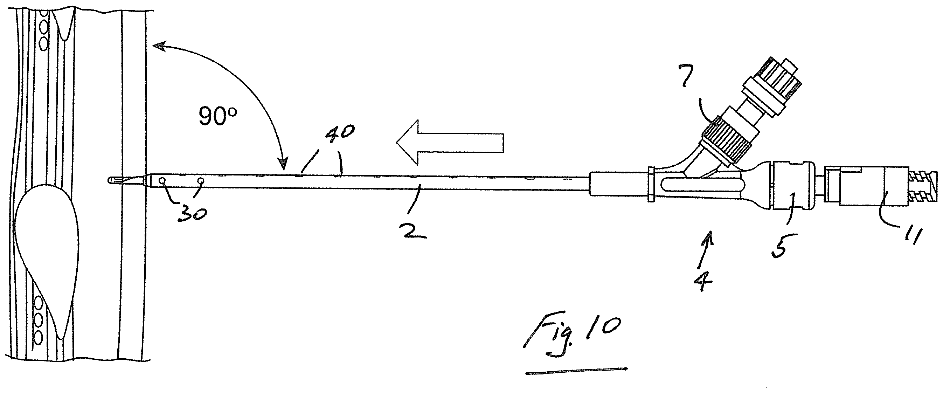

Fig. 10 shows the correct insertion method for the device just above the upper border of the third rib (i.e. into the second intercostal space) in the anterior mid-clavicular line, to avoid the intercostal neurovascular bundle for the management of a pneumothroax

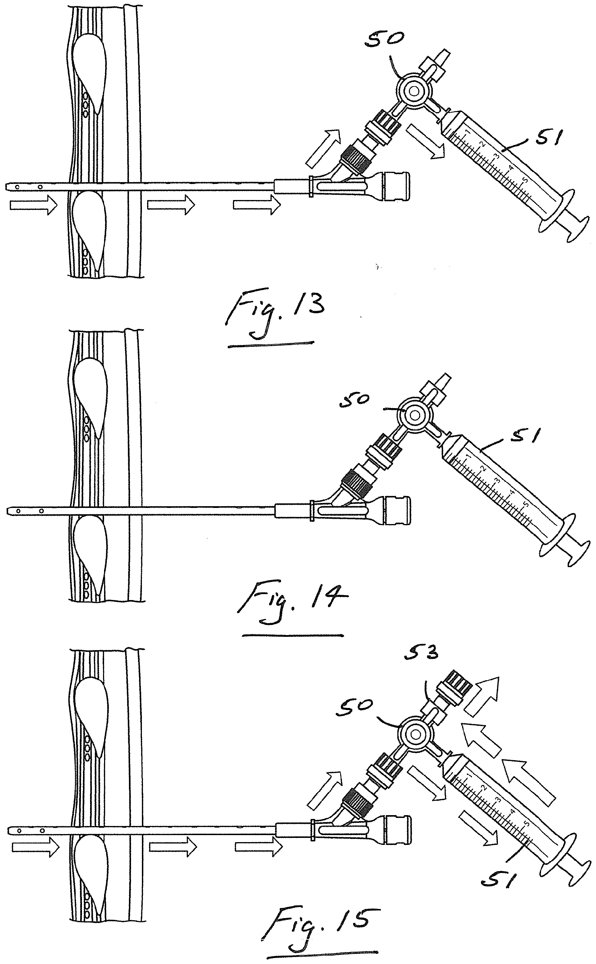

Fig. 13 shows the inserted device with the Veress needle 10 withdrawn and a three way tap 50 attached and a syringe 51 attached to another port of the three way tap 50. In practice, the flexible nature of the cannula allows the taping of the device to the patient's skin surface.

Fig. 14 shows the inserted device with the Veress needle 10 withdrawn with a three way tap 50 attached and a syringe 51 attached to another port of the three way tap with the final tap closed. In this embodiment of the invention, if the syringe plunger 51 is withdrawn it will draw fluid and/or gases into the syringe in the direction shown by the blacked out arrows.

Fig. 15 shows an alternative arrangement to Fig. 12 which comprises the addition of low pressure one way cracking valve 53 to the final port of the three way tap 50. On withdrawal of the syringe plunger 51 fluid and/or gases is withdrawn from the pneumothorax or effusion into the syringe 51 in the direction shown by the blacked out arrows. Subsequent depressing of the syringe 51 pumps the fluid and/or gas out through the one way low pressure valve 53 in the direction indicated by the smaller arrows, thereby providing a simple pump system for effusion and pneumothorax treatment.

The invention provides a quick, easy, single step insertion device that can be used to manage conditions such pneumothoraxes and effusions particularly by providing a failsafe means to expire fluids and/or gas from the body. Moreover the invention provides for a pump system for the removal of fluid and/or gas from the body in a system that is more simplistic than any currently conceived.

### Example 1 - Skin and tissue penetration

The device of the invention successfully penetrated skin/muscle penetration using an uncooked pork belly sample. Uncooked pork belly provides a good human skin/muscle substitute due to its outer tough skin and multiple tissue layers.

### Example 2 - Freshly thawed cadaver pleural cavity penetration

A cadaver study was performed with the invention. The device was tested for insertion into a freshly thawed non-embalmed cadaver. The study mimicked the placement of the device for the management of a tension pneumothorax. The device insertion site was located and insertion with the device was as described above. The device successfully penetrated the skin surface.

### Example 3 - Embalmed cadaver fluid expiration test

Example 2 was repeated using an embalmed cadaver. After insertion, embalming fluid escaped through the Veress needle on entry and after Veress needle withdrawal the fluid escaped through the side port one way crack valve but not through the self-sealing bung. This was representative of the in vivo fluid/gas functionality of the device.

### Example 4 - Aspiration through three way tap

The device was attached to a three way tap as shown in Fig 14 with a syringe also attached to the three way tap. The device cannula was inserted into a beaker of water with the Veress needle removed instead of into the pleural cavity as specifically shown in Fig 14. This set up was intended to mimic the device operation in a pleural effusion or a simple pneumothorax case. The third port on the three way tap was closed and the plunger in the syringe was withdrawn. Water was drawn from the beaker through the cannula and subsequently through the low pressure one way cracking valve into the three way tap and finally into the syringe. This confirmed successful device characteristics for the withdrawing of fluid from the body in such medical management cases as simple pneumothorax or pleural effusion.

### Example 5 - Fluid pumping

In a similar experimental set-up to Example 4 a low pressure one way cracking valve was added to the final port of the three way tap as can be seen in Figure 15. The low pressure one way cracking valve was purchased from Promepla SA (part number PBB01050). With this valve it is possible to orientate it so that it is possible to control which direction the fluid and/or gases pass through it.

As with Example 4, water was drawn from the beaker through the cannula and subsequently through the low pressure one way cracking valve into the three way tap and finally into the syringe. Subsequently when the syringe plunger was depressed, as water cannot pass back through the one way cracking valve on the side port of the device hub it is expelled through the other low pressure one way cracking valve to a collection vessel as that valve is orientated to let fluid out but no air or fluid in to the three way tap. Subsequent withdrawal of the syringe plunger takes up more water, with subsequent depression of the plunger pumping more water from the container. This example demonstrates the simple adaptation of the invention into a manual pump that can be used for the management of certain medical conditions.

Although the disclosure hereof is detailed and exact to enable those skilled in the art to practice the invention, the physical embodiments herein disclosed merely exemplify the invention that may be embodied in other ways. While the preferred embodiment has been described the details may be changed without departing from the invention.

Modifications and additions can be made to the embodiments of the invention described herein without departing from the scope of the invention. For example, while the embodiments described herein refer to particular features, the invention includes embodiments having different combinations of features. The invention also includes embodiments that do not include all of the specific features described.

### References

1. Barton ED, et al. P. Prehospital needle aspiration and tube thoracostomy in trauma victims: a six year experience with aeromedical crews. J Emerg Med. 1995;13:155-163.
2. Davis DP, et al. The safety and efficacy of prehospital needle and tube thoracostomy by aeromedical personnel. Prehosp Emerg Care. 2005;9:191-197.
3. Zengerink, I er al. Needle Thoracostomy in the Treatment of a Tension Pneumothorax in Trauma Patients: What Size Needle? J Trauma. 2008;64:111-114.
4. Ball, CG et al. Thoracic needle decompression for tension pneumothorax: clinical correlation with catheter length. J can chir, 2010 Vol. 53, No 3
5. Harcke, HT et al. Chest Wall Thickness in Military Personnel: Implications for Needle Thoracentesis in Tension Pneumothorax. Journal of Special Operations Medicine 2008 Volume 8, Edition 2.
6. R Jones, J Hollingsworth. Tension pneumothoraces not responding to needle Thoracocentesis. Emerg Med J 2002;19:176-177
7. Groves and Parekh. Death Following Thoracentesis: Investigating The Cause. The Internet Journal of Emergency and Intensive Care Medicine
8. The prehospital management of chest injuries a consensus statement. Faculty of Pre-hospital Care, Royal College of Surgeons of Edinburgh
9 Rawlins R, Brown KM, Carr CS, et al. Life threatening haemorrhage after anterior needle aspiration of pneumothoraces. A role for lateral needle aspiration in emergency decompression of spontaneous pneumothorax. Emerg Med J 2003;20:383-4.
10. Seneff MG, Corwin RW, Gold LH, et al. Complications associated with thoracocentesis. Chest 1986;90:97-100.
11. Carney M, Ravin CE. Intercostal artery laceration during thoracocentesis. Chest 1979;75:520-2.
12. Butler KL, Best IM, Weaver WL, et al. Pulmonary artery injury and cardiac tamponade after needle decompression of a suspected tension pneumothorax. J Trauma 2003;54:610-11.

## Claims

1. A single step body insertion device for removal of fluid from a body comprising a cannula (1) having:-
a cannula shaft (2) having a longitudinal axis along a length thereof;
a tapered tip (3) at the distal end of the cannula shaft; the tapered tip (3) including an inner surface forming a first frustoconical surface concentrically around the longitudinal axis and an outer surface forming a second frustoconical surface concentrically around the longitudinal axis and first frustoconical surface, and the tapered tip having an inner diameter (IDt) along at least a portion of the tip that is less than the inner diameter (IDb) of a main body of the cannula shaft; and
a hub (4) at the proximal end of the cannula shaft, the hub having:-
a sealing valve (6) aligned with the longitudinal axis of the cannula shaft (2) for sealingly engaging a needle (12) which is adapted to extend through the cannula to penetrate the skin surface to facilitate delivery of the distal end of the cannula for fluid to be removed from the body; and
a side port (7) with a one-way low pressure cracking valve having a cracking pressure of ≤ 12mbar and a backward pressure of up to 6 bar which permits flow of fluid through the valve (8) only in the proximal direction,
wherein the cannula shaft (2) is flexible and kink resistant, the main body of the cannula shaft is of a uniform thickness and the wall thickness progressively reduces inwardly distally along the distal tip of the cannula, and
wherein the cannula shaft (2) is of an aliphatic polyether-based thermoplastic polyurethane with a shore hardness of about 63D.

2. A device as claimed in claim 1 wherein the cannula shaft (2) is radiopaque, the shaft preferably contains a radiopaque material, the radiopaque material may be barium sulphate, barium sulphate may be present at a loading of about 20% by weight in the cannula of the device.

3. A device as claimed in claim 1 or 2 wherein the inner diameter (IDt) along the length of the tapered tip (3) is less than the inner diameter (IDb) of the main body of the cannula shaft, the difference between IDb and IDt may be less than 0.4mm, preferably less than 0.2mm, preferably approximately 0.16mm.

4. A device as claimed in any of claims 1 to 3 wherein the taper angle of the outer surface of the distal tip (3) is from 4° to 13°, the taper angle may be from 7° to 11°, the taper angle may be approximately 9°.

5. A device as claimed in any of claims 1 to 4 wherein the length of the tapered distal tip (3) is less than 10mm, the length of the tapered distal tip may be about 4mm.

6. A device as claimed in any of claims 1 to 5 wherein the length of the cannula shaft (2) is at least 100mm, the length of the cannula shaft (2) may be from 100 to 150mm, the length of the cannula shaft may be about 115mm.

7. A device as claimed in any of claims 1 to 6 wherein the cannula shaft (2) has an outer diameter in the range of from 2 to 5mm, the cannula shaft may have an outer diameter in the range of from 3 to 4mm, the cannula shaft may have an outer diameter of about 3.5mm.

8. A device as claimed in any of claims 1 to 7 wherein the main body of the cannula shaft (2) has a wall thickness in the range of from 0.5mm to 1mm, the main body of the cannula shaft may have a wall thickness of about 0.7mm.

9. A device as claimed in any of claims 1 to 8 wherein the main body of the cannula shaft (2) has at least one opening (30) in the wall thereof adjacent to the distal tip (3) to provide a further pathway for fluid entry into the cannula shaft.

10. A device as claimed in any of claims 1 to 9 wherein the cannula shaft (2) has indicia thereon to indicate the depth of penetration of the cannula shaft into the body.

11. A device as claimed in claim 10 wherein at least some of the indicia are luminous.

12. A device as claimed in any of claims 1 to 11 wherein the side port comprises a connector for mounting of ancillary components to the side port, a three way tap (50) for attachment to the side port, and a syringe (51) for attachment to one of the ports of the three way tap.

## Patentansprüche

1. Einstufige Körpereinführungsvorrichtung zur Beseitigung von Flüssigkeit aus einem Körper, umfassend eine Kanüle (1), die Folgendes aufweist:
einen Kanülenschaft (2), der eine Längsachse entlang seiner Länge aufweist;
eine konische Spitze (3) an dem distalen Ende des Kanülenschafts; wobei die konische Spitze (3) eine Innenfläche, die eine erste kegelstumpfförmige Fläche konzentrisch um die Längsachse bildet, und eine Außenfläche, die eine zweite kegelstumpfförmige Fläche konzentrisch um die Längsachse und die erste kegelstumpfförmige Fläche bildet, beinhaltet und die konische Spitze einen Innendurchmesser (IDt) entlang mindestens eines Abschnitts der Spitze aufweist, der kleiner als der Innendurchmesser (IDb) eines Hauptkörpers des Kanülenschafts ist; und
einen Ansatz (4) an dem proximalen Ende des Kanülenschafts, wobei der Ansatz Folgendes aufweist:
ein an der Längsachse des Kanülenschafts (2) ausgerichtetes Dichtungsventil (6) zum abdichtenden Ineingriffbringen einer Nadel (12), die zum Erstrecken durch die Kanüle zum Durchdringen der Hautoberfläche beschaffen ist, um die Abgabe des distalen Endes der Kanüle zum Beseitigen von Flüssigkeit aus dem Körper zu erleichtern; und
einen Seitenport (7) mit einem Einweg-Niederdruck-Abreißventil, das einen Abreißdruck von ≤ 12 mbar und einen Gegendruck von bis zu 6 bar aufweist, was einen Flüssigkeitsfluss durch das Ventil (8) nur in der proximalen Richtung zulässt,
wobei der Kanülenschaft (2) flexibel und knickfest ist, der Hauptkörper des Kanülenschafts eine gleichmäßige Dicke aufweist und die Wandstärke entlang der distalen Spitze der Kanüle nach innen distal nach und nach abnimmt, und
wobei der Kanülenschaft (2) aus einem thermoplastischen Polyurethan auf der Basis eines aliphatischen Polyethers mit einer Shore-Härte von etwa 63D ist.

2. Vorrichtung nach Anspruch 1, wobei der Kanülenschaft (2) röntgenundurchlässig ist, der Schaft vorzugsweise ein röntgenundurchlässiges Material enthält, das röntgenundurchlässige Material Bariumsulfat sein kann und Bariumsulfat mit einer Beladung von etwa 20 Gew.-% in der Kanüle der Vorrichtung vorhanden sein kann.

3. Vorrichtung nach Anspruch 1 oder 2, wobei der Innendurchmesser (IDt) entlang der Länge der konischen Spitze (3) kleiner als der Innendurchmesser (IDb) des Hauptkörpers des Kanülenschafts ist, wobei die Differenz zwischen IDb und IDt weniger als 0,4 mm, vorzugsweise weniger als 0,2 mm, vorzugsweise etwa 0,16 mm betragen kann.

4. Vorrichtung nach einem der Ansprüche 1 bis 3, wobei der Kegelwinkel der Außenfläche der distalen Spitze (3) 4° bis 13° beträgt, der Kegelwinkel 7° bis 11° betragen kann, der Kegelwinkel 9° betragen kann.

5. Vorrichtung nach einem der Ansprüche 1 bis 4, wobei die Länge der konischen distalen Spitze (3) weniger als 10 mm beträgt, wobei die Länge der konischen distalen Spitze etwa 4 mm betragen kann.

6. Vorrichtung nach einem der Ansprüche 1 bis 5, wobei die Länge des Kanülenschafts (2) mindestens 100 mm beträgt, die Länge des Kanülenschafts (2) 100 bis 150 mm betragen kann, die Länge des Kanülenschafts 115 mm betragen kann.

7. Vorrichtung nach einem der Ansprüche 1 bis 6, wobei der Kanülenschaft (2) einen Außendurchmesser in dem Bereich von 2 bis 5 mm aufweist, der Kanülenschaft einen Außendurchmesser im Bereich von 3 bis 4 mm aufweisen kann, der Kanülenschaft einen Außendurchmesser von etwa 3,5 mm aufweisen kann.

8. Vorrichtung nach einem der Ansprüche 1 bis 7, wobei der Hauptkörper des Kanülenschafts (2) eine Wandstärke in dem Bereich von 0,5 mm bis 1 mm aufweist, der Hauptkörper des Kanülenschafts eine Wandstärke von etwa 0,7 mm aufweisen kann.

9. Vorrichtung nach einem der Ansprüche 1 bis 8, wobei der Hauptkörper des Kanülenschafts (2) mindestens eine Öffnung (30) in der Wand davon neben der distalen Spitze (3) aufweist, um einen weiteren Weg für Flüssigkeitseintritt in den Kanülenschaft bereitzustellen.

10. Vorrichtung nach einem der Ansprüche 1 bis 9, wobei an dem Kanülenschaft (2) Markierungen angebracht sind, um die Eindringtiefe des Kanülenschafts in den Körper anzuzeigen.

11. Vorrichtung nach Anspruch 10, wobei mindestens einige der Markierungen leuchtend sind.

12. Vorrichtung nach einem der Ansprüche 1 bis 11, wobei der Seitenport einen Konnektor zum Anbringen von Zusatzkomponenten am Seitenport, einen Dreiwegehahn (50) zur Befestigung an dem Seitenport und eine Spritze (51) zur Befestigung an einem der Ports des Dreiwegehahns umfasst.

## Revendications

1. Dispositif d'insertion corporelle en une seule étape pour l'élimination de fluide dans un corps comprenant une canule (1) ayant : -
une tige de canule (2) ayant un axe longitudinal sur une longueur de celle-ci ;
une pointe effilée (3) à l'extrémité distale de la tige de canule ; la pointe effilée (3) comprenant une surface intérieure formant une première surface tronconique concentriquement autour de l'axe longitudinal et une surface extérieure formant une seconde surface tronconique concentriquement autour de l'axe longitudinal et une première surface tronconique, et la pointe effilée ayant un diamètre intérieur (IDt) le long d'au moins une partie de la pointe qui est inférieure au diamètre intérieur (IDb) d'un corps principal de la tige de canule ; et
un moyeu (4) à l'extrémité proximale de la tige de canule, le moyeu ayant : -
une valve d'étanchéité (6) alignée avec l'axe longitudinal de la tige de canule (2) pour faire entrer en prise de manière étanche une aiguille (12) qui est adaptée pour s'étendre à travers la canule pour pénétrer la surface de la peau afin de faciliter l'administration de l'extrémité distale de la canule pour fluide devant être retirée du corps ; et
un orifice latéral (7) avec une soupape de craquage basse pression unidirectionnelle ayant une pression de craquage ≤ 12 mbar et une contre-pression allant jusqu'à 6 bar qui permet l'écoulement de fluide à travers la soupape (8) uniquement dans la direction proximale,
dans lequel la tige de canule (2) est flexible et résistante au vrillage, le corps principal de la tige de canule est d'une épaisseur uniforme et l'épaisseur de paroi se réduit progressivement vers l'intérieur de manière distale le long de la pointe distale de la canule, et
dans laquelle la tige de canule (2) est en polyuréthane thermoplastique à base de polyéther aliphatique avec une dureté shore d'environ 63D.

2. Dispositif selon la revendication 1, dans lequel la tige de canule (2) est radio-opaque, la tige contient de préférence un matériau radio-opaque, le matériau radio-opaque peut être du sulfate de baryum, du sulfate de baryum peut être présent à une charge d'environ 20% en poids dans la canule du dispositif.

3. Dispositif selon la revendication 1 ou 2, dans lequel le diamètre intérieur (IDt) sur la longueur de la pointe effilée (3) est inférieur au diamètre intérieur (IDb) du corps principal de la tige de canule, la différence entre IDb et IDt peut être inférieure à 0,4 mm, de préférence inférieure à 0,2 mm, de préférence d'environ 0,16 mm.

4. Dispositif selon l'une quelconque des revendications 1 à 3, dans lequel l'angle de conicité de la surface extérieure de la pointe distale (3) est de 4° à 13°, l'angle de conicité peut être de 7° à 11°, l'angle de conicité peut être d'environ 9°.

5. Dispositif selon l'une quelconque des revendications 1 à 4, dans lequel la longueur de la pointe distale effilée (3) est inférieure à 10 mm, la longueur de la pointe distale effilée peut être d'environ 4 mm.

6. Dispositif selon l'une quelconque des revendications 1 à 5, dans lequel la longueur de la tige de canule (2) est d'au moins 100 mm, la longueur de la tige de canule (2) peut être de 100 à 150 mm, la longueur de la tige de canule peut être d'environ 115 mm.

7. Dispositif selon l'une quelconque des revendications 1 à 6, dans lequel la tige de canule (2) a un diamètre extérieur dans la plage de 2 à 5 mm, la tige de canule peut avoir un diamètre extérieur dans la plage de 3 à 4 mm, la tige de canule peut avoir un diamètre extérieur d'environ 3,5 mm.

8. Dispositif selon l'une quelconque des revendications 1 à 7, dans lequel le corps principal de la tige de canule (2) a une épaisseur de paroi dans la plage de 0,5 mm à 1 mm, le corps principal de la tige de canule peut avoir une épaisseur de paroi d'environ 0,7 mm.

9. Dispositif selon l'une quelconque des revendications 1 à 8, dans lequel le corps principal de la tige de la canule (2) a au moins une ouverture (30) dans sa paroi adjacente à la pointe distale (3) pour fournir une autre voie pour une entrée de fluide dans la tige de canule.

10. Dispositif selon l'une quelconque des revendications 1 à 9, dans lequel la tige de canule (2) porte des indices pour indiquer la profondeur de pénétration de la tige de canule dans le corps.

11. Dispositif selon la revendication 10, dans lequel au moins certains parmi les indices sont lumineux.

12. Dispositif selon l'une quelconque des revendications 1 à 11, dans lequel l'orifice latéral comprend un connecteur pour le montage de composants auxiliaires sur l'orifice latéral, un robinet à trois voies (50) pour la fixation à l'orifice latéral, et une seringue (51) pour la fixation à l'un des ports du robinet à trois voies.
